# EUROPEAN PATENT APPLICATION

(11) **EP 4 596 026 A1**
(43) Date of publication of application: **06.08.2025**
(21) Application number: 23872792.9
(22) Date of filing: 11.08.2023
(51) Int. Cl.: A61N 1/36, A61N 1/05

(54) **DEEP BRAIN STIMULATION DEVICE, AND CONTROL METHOD THEREOF**

(30) Priority: 29.09.2022 KR 20220124233
(71) Applicant: Seoul National University R&DB Foundation, Seoul 08826 (KR); Brain & Beyonds Co., Ltd., Seoul 03122 (KR)
(72) Inventor: PAEK, Sun Ha, Seoul 05274 (KR); PARK, Soon Beom, Seoul 05274 (KR)
(74) Representative: Algemeen Octrooi- en Merkenbureau B.V.
(86) International application number: PCT/KR2023/011977
(87) International publication number: WO 2024/071669

(57) **Abstract**

According to a deep brain stimulation device and a control method thereof according to one embodiment of the present invention, two electrodes are inserted into a deep brain nucleus and supplied with high-frequency current in order to treat brain and neural diseases such as Parkinson's disease, tremor, myodystonia, Alzheimer's dementia, epilepsy, obsessive compulsive disorder, depression, pain, drug addiction, and post-traumatic syndrome, wherein the two electrodes are not supplied with current at the same time, thus making it possible to extend the life of a battery while achieving treatment effects that are the same as or superior to the prior art.

## Description

### [Technical Field]

The present disclosure relates to a deep brain stimulation device and a deep brain stimulation method using the same, and more particularly, to a deep brain stimulation device and a control method thereof, in which two electrodes are inserted into a deep brain nucleus and supplied with high-frequency current in order to treat brain and neural diseases such as Parkinson's disease, tremor, myodystonia, Alzheimer's dementia, epilepsy, obsessive compulsive disorder, depression, pain, drug addiction, and post-traumatic syndrome, wherein the two electrodes are not supplied with current at the same time, thus making it possible to extend the life of a battery while achieving treatment effects that are at least equivalent to or superior to those of the prior art.

### [Background Art]

A group of diseases that clinically exhibit symptoms of Parkinson's disease, such as bradykinesia, rigidity, and resting tremor, is collectively called parkinsonism, including Parkinson's disease, secondary parkinsonism caused by infections, drugs, toxic substances, or brain damage due to cerebrovascular disease, and Parkinson-plus syndrome, which is atypical parkinsonism with conditions such as multiple system atrophy, progressive supranuclear palsy, and corticobasal degeneration. Parkinson's disease, first described by James Parkinson in 1817, is the second most common degenerative disease of the central nervous system after Alzheimer's disease and accounts for approximately 80% of patients with Parkinson's syndrome. It is known that symptoms of Parkinson's disease develop as degenerative changes in dopaminergic neurons, which characteristically originate from the substantia nigra pars compacta (SNc) and extend their neurites to the corpus striatum, progress gradually. However, the cause or mechanism of Parkinson's disease has not yet been identified, so Parkinson's disease is a degenerative disease of the central nervous system for which no preventive or curative treatment can be expected. It is well known that dopamine deficiency caused by the gradual loss of dopamine cells in the substantia nigra pars compacta, leads to the symptoms of Parkinson's disease, and treatment methods have been attempted to alleviate the symptoms. Treatments for alleviating the symptoms of Parkinson's disease include drug therapy and surgical treatment, and these two treatments have been complementary in alleviating patients' symptoms. Since the late 1940s, surgical treatment has been promoted by advances in stereotactic surgery techniques, but with the development of L-dopa drugs in the late 1960s, surgical treatment, which had a high complication rate, began to decline. Other drug treatments for Parkinson's disease include dopamine agonists such as apomorphine, bromocriptine, pergolide, paramipexole, ropinirole, carbergoline, and lisuride, anticholinergics such as trihexyphenidyl (Artane), benztropin (Cogentin), and biperidin (Akinetone), catechol-O-methyl transferase (COMT) inhibitors such as amantadine, tolcapone, and entacapone, which are antiviral drugs, and monoamine oxidase-B (MAO-B) inhibitors such as selegiline (deprenyl). However, dyskinesia, motor fluctuations, and other complications (gastrointestinal disorders, mental disorders) that occur after long-term overuse of Parkinson's disease medications including L-dopa, have made it difficult to alleviate patients' symptoms with drug therapy alone. On the other hand, advances in computer science, the development of new brain imaging technologies such as brain computed tomography and brain magnetic resonance imaging, and advances in surgical techniques have made stereotactic surgery more accurate and less complicated, and have led to a wealth of electrophysiological knowledge in patients with Parkinson's disease and animal models of Parkinson's disease. Based on this, surgical treatment for patients with Parkinson's disease began to experience a revival in the late 1980s, as it effectively alleviated the side effects of long-term use of Parkinson's disease medications. Initially, surgery was performed to destroy a deep brain nucleus region where electrophysiological signals were abnormally increased in patients with Parkinson's disease using electrocautery. However, in the late 1980s, Benabid discovered that stimulating the deep brain nucleus region with high-frequency electric current could achieve the same therapeutic effect as electrocautery without destroying tissue, and this approach was then applied in treatment. In 1997, deep brain stimulation (DBS) for tremor was approved by the US Food and Drug Administration, and in 2002, DBS for Parkinson's disease was approved by the US Food and Drug Administration. Since then, many patients with Parkinson's disease have received deep brain stimulation. In Korea, the Korean Food and Drug Administration approved deep brain stimulation for tremor and Parkinson's disease in 2000, and since deep brain stimulation became covered by insurance in January 2005, many patients with Parkinson's disease in Korea have been able to receive deep brain stimulation. In recent years, the sustained-release administration of L-dopa using L-dopa patches or L-dopa gastrointestinal pumps has been developed and is helpful in some patients, but in most cases where dyskinesia, motor fluctuations, and other complications (gastrointestinal disorders, mental disorders) occur after long-term overuse of Parkinson's disease medications, deep brain stimulation is performed. In recent years, this deep brain stimulation has also been widely used in patients with brain and neural diseases such as tremor, myodystonia, Alzheimer's dementia, epilepsy, obsessive compulsive disorder, depression, pain, drug addiction, post-traumatic syndrome, etc., including Parkinson's disease.

### [Disclosure]

### [Technical Problem]

A technical problem to be solved by a deep brain stimulation device and a control method thereof according to an embodiment of the technical idea of the present disclosure is to provide a deep brain stimulation device comprising a first stimulation electrode configured to be located at a first location of a deep brain nucleus, a second stimulation electrode configured to be located at a second location of the deep brain nucleus, and a stimulator supplying currents to the first stimulation electrode and the second stimulation electrode, wherein the stimulator is controlled so as not to supply the currents to the first stimulation electrode and the second stimulation electrode simultaneously.

A technical problem to be solved by a deep brain stimulation device and a control method thereof according to an embodiment of the technical idea of the present disclosure is to provide a deep brain stimulation device wherein the stimulator is controlled to supply the current to only any one of the first and second stimulation electrodes.

A technical problem to be solved by a deep brain stimulation device and a control method thereof according to an embodiment of the technical idea of the present disclosure is to provide a deep brain stimulation device wherein the stimulator is controlled to alternately supply the currents to each of the first and second stimulation electrodes.

A technical problem to be solved by a deep brain stimulation device and a control method thereof according to an embodiment of the technical idea of the present disclosure is to provide a deep brain stimulation device wherein the stimulator comprises a first stimulator connected to the first stimulation electrode and supplying the current to the first stimulation electrode, and a second stimulator connected to the second stimulation electrode and supplying the current to the second stimulation electrode, wherein the first stimulator and the second stimulator are controlled so as not to supply the currents to the first stimulation electrode and the second stimulation electrode simultaneously.

A technical problem to be solved by a deep brain stimulation device and a control method thereof according to an embodiment of the technical idea of the present disclosure is to provide a deep brain stimulation device wherein the current supplied to the first stimulation electrode and the current supplied to the second stimulation electrode have the same magnitude.

A technical problem to be solved by a deep brain stimulation device and a control method thereof according to an embodiment of the technical idea of the present disclosure is to provide a deep brain stimulation device wherein a first current supply time and a second current supply time are the same as each other.

A technical problem to be solved by a deep brain stimulation device and a control method thereof according to an embodiment of the technical idea of the present disclosure is to provide a deep brain stimulation device wherein after the current is supplied to the first stimulation electrode, the current is supplied to the second stimulation electrode, when a predetermined rest time has elapsed.

A technical problem to be solved by a deep brain stimulation device and a control method thereof according to an embodiment of the technical idea of the present disclosure is to provide a deep brain stimulation device wherein the first current supply time and the second current supply time are the same as each other.

A technical problem to be solved by a deep brain stimulation device and a deep brain stimulation method using the same according to an embodiment of the technical idea of the present disclosure is not limited to those for solving the problems mentioned above, and other problem not mentioned will be clearly understood by those skilled in the art from the description below.

### [Technical Solution]

According to an embodiment of the present disclosure, a deep brain stimulation device may comprise a first stimulation electrode configured to be located at a first location of a deep brain nucleus; a second stimulation electrode configured to be located at a second location of the deep brain nucleus; and a stimulator connected to the first stimulation electrode and the second stimulation electrode and supplying the current to the first stimulation electrode and the second stimulation electrode, wherein the stimulator may be controlled so as not to supply the currents to the first stimulation electrode and the second stimulation electrode simultaneously. This means that the currents are controlled so as not to be supplied to the first and second stimulation electrodes simultaneously, and means that the current may be continuously supplied to only any one of the first and second stimulation electrodes or the currents may be alternately supplied to each of the first and second stimulation electrodes.

The stimulator may perform a current supply method comprising: not supplying the current to the second stimulation electrode when supplying the current to the first stimulation electrode during a first current supply time; and not supplying the current to the first stimulation electrode and supplying the current to the second stimulation electrode during a second current supply time, when the first current supply time has elapsed.

The current supplied to the first stimulation electrode and the current supplied to the second stimulation electrode may have the same magnitude.

The current supply method may further comprise: allowing the stimulator to have a predetermined rest time before supplying the current to the second stimulation electrode, after supplying the current to the first stimulation electrode, wherein during the predetermined rest time, no current may be supplied to both the first stimulation electrode and the second stimulation electrode.

According to another embodiment of the present disclosure, a deep brain stimulation device may comprise a first stimulation electrode configured to be located at a first location of a deep brain nucleus; a second stimulation electrode configured to be located at a second location of the deep brain nucleus; a first stimulator configured to be connected to the first stimulation electrode and supplying the current to the first stimulation electrode; and a second stimulator configured to be connected to the second stimulation electrode and supplying the current to the second stimulation electrode, wherein the first stimulator and the second stimulator may be controlled so as not to supply the currents to the first stimulation electrode and the second stimulation electrode simultaneously. This means that the currents are controlled so as not to be supplied to the first and second stimulation electrodes simultaneously, and means that the current may be continuously supplied to only any one of the first and second stimulation electrodes or the currents may be alternately supplied to each of the first and second stimulation electrodes.

In the deep brain stimulation device, a current supply method may be performed, the method comprising: the second stimulator not supplying the current to the second stimulation electrode when the first stimulator supplies the current to the first stimulation electrode during a first current supply time; and the first stimulator not supplying the current to the first stimulation electrode and the second stimulator supplying the current to the second stimulation electrode during a second current supply time, when the first current supply time has elapsed.

The current supplied to the first stimulation electrode and the current supplied to the second stimulation electrode may have the same magnitude, and the first current supply time and the second current supply time may be the same as each other.

The deep brain stimulation device may allow the second stimulator to supply the current to the second stimulation electrode during the second current supply time after a predetermined rest time, after the first stimulator supplies the current to the first stimulation electrode during the first current supply time.

According to an embodiment of the present disclosure, a control method of deep brain stimulation device may comprise: supplying the current to a first stimulation electrode located at a first location of the deep brain nucleus during a first current supply time; and supplying the current to a second stimulation electrode located at a second location of the deep brain nucleus during a second current supply time, after the first current supply time, wherein the current may be controlled so as not to be supplied to the second stimulation electrode while the current is supplied to the first stimulation electrode, and the current may be controlled so as not to be supplied to the first stimulation electrode while the current is supplied to the second stimulation electrode. This means that the currents are not supplied to the first and second stimulation electrodes simultaneously, and means that the current may be continuously supplied to only any one of the first and second stimulation electrodes or the currents may be alternately supplied to each of the first and second stimulation electrodes.

The current supplied to the first stimulation electrode and the current supplied to the second stimulation electrode may have the same magnitude, and the first current supply time and the second current supply time may be the same as each other.

After the current is supplied to the first stimulation electrode, the current may be supplied to the second stimulation electrode, when a predetermined rest time has elapsed.

### [Advantageous Effects]

According to a deep brain stimulation device and a control method thereof according to an embodiment of the technical idea of the present disclosure, two electrodes are inserted into a deep brain nucleus and supplied with high-frequency current in order to treat brain and neural diseases such as Parkinson's disease, tremor, myodystonia, Alzheimer's dementia, epilepsy, obsessive compulsive disorder, depression, pain, drug addiction, and post-traumatic syndrome, wherein the two electrodes are controlled not to be supplied with current at the same time, thus making it possible to extend the life of a battery while achieving treatment effects that are at least equivalent to those of the prior art.

The effects that can be achieved by a deep brain stimulation device and a control method thereof according to an embodiment of the technical idea of the present disclosure are not limited to those mentioned above, and other effects not mentioned will be clearly understood by those skilled in the art from the following description.

### [Description of Drawings]

For a more complete understanding of the drawings cited herein, a brief description of each drawing is provided.
FIG. 1 is a drawing schematically illustrating a deep brain stimulation device according to an embodiment of the technical idea of the present disclosure.
FIG. 2 is a drawing schematically illustrating an installation state of a deep brain stimulation device according to an embodiment of the technical idea of the present disclosure.
FIG. 3 is a drawing schematically illustrating a deep brain stimulation method according to an embodiment of the technical idea of the present disclosure.
FIG. 4 is a drawing schematically illustrating a deep brain stimulation device according to another embodiment of the technical idea of the present disclosure.
FIG. 5 is a schematic drawing schematically illustrating an installation state of a deep brain stimulation device according to another embodiment of the technical idea of the present disclosure.

### [Best Mode]

The technology disclosed herein is subject to various modifications and may have many different embodiments. Therefore, specific embodiments are illustrated in the drawings and will be described in detail through the detailed description. However, this is not intended to limit the technology disclosed herein to any particular embodiment, and it should be understood that the technology disclosed herein includes all modifications, equivalents, or substitutes included in the spirit and scope of the technology disclosed herein.

In describing the technology disclosed herein, if it is determined that a specific description of a related known technology may unnecessarily obscure the gist of the technology disclosed herein, the detailed descriptions thereof will be omitted. In addition, the numbers (e.g., first, second, etc.) used in the description herein are merely identifiers to distinguish one component from another component.

Further, whenever a component is referred to herein as being "connected to" or "coupled to" another component, it is to be understood that any of the above components may be directly connected or coupled to the above other components, but may also be connected or coupled to another component intervening between them, unless specifically stated to the contrary.

In addition, the components expressed as "~part" herein may be two or more components combined into a single component, or a single component may be divided into two or more components with more detailed functions. Also, each of the components described below may additionally perform, in addition to the primary functions for which they are responsible, some or all of the functions for which other components are responsible. Likewise, some of the primary functions for which each of the components is responsible may be exclusively performed by other components.

The expressions "first," "second," and the like used in various embodiments may modify various components, regardless of order and/or importance, and do not limit those components. For example, the first component may be named interchangeably with the second component, and similarly, the second component may be named interchangeably with the first component, without departing from the scope of the technology disclosed herein.

Hereinafter, a deep brain stimulation device and a control method thereof according to a preferred embodiment will be described in detail.

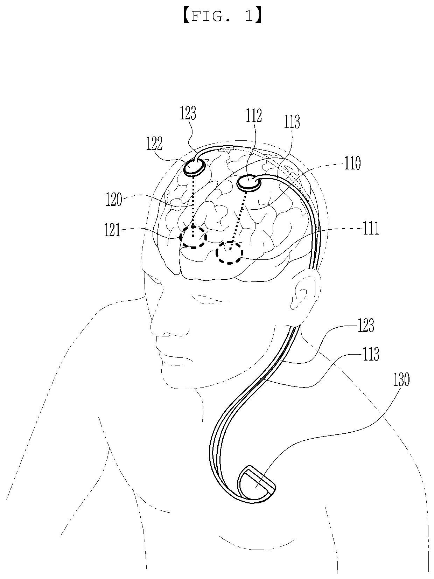

FIG. 1 is a diagram schematically illustrating a deep brain stimulation device according to an embodiment of the technical idea of the present disclosure.

A deep brain stimulation device according to an embodiment of the technical idea of the present disclosure may comprise a first stimulation electrode 110, a second stimulation electrode 120, and a stimulator 130.

The first stimulation electrode 110 may be located at a first location 111 of a deep brain nucleus, and the second stimulation electrode 120 may be located at a second location 121 of the deep brain nucleus. For example, the first stimulation electrode 110 and the second stimulation electrode 120 may be long rod-shaped electrodes made of platinum or iridium, and may perform the function of applying electrical stimulation to the first location 111 and the second location 121 of the deep brain nucleus, respectively.

The first stimulation electrode 110 and the second stimulation electrode 120 may be fixed to a perforated skull through a first fixing part 112 and a second fixing part 122, respectively. The first stimulation electrode 110 and the second stimulation electrode 120 are connected to a stimulator 130 by a subcutaneous passage through a first extension line 113 and a second extension line 123, respectively, to form a pair of electrodes.

The first location 111 and the second location 121 may be the ventral intermediate nucleus of the thalamus, the medial globus pallidus, and the subthalamic nucleus, etc. in the case of Parkinson's disease. The first location 111 and the second location 121 may be located so as to be symmetrical on both sides with respect to the center of the brain.

The ventral intermediate nucleus of the thalamus may be selected as the first location 111 and the second location 121 in patients with tremor of various causes, including Parkinson's disease tremor.

The medial globus pallidus and the subthalamic nucleus may be selected as the first location 111 and the second location 121 to alleviate symptoms in patients with Parkinson's disease receiving long-term L-dopa administration.

Stimulation of the ventral intermediate nucleus of the thalamus has no effect on alleviating symptoms of Parkinson's disease other than tremor, and stimulation of the medial globus pallidus or the subthalamic nucleus alleviates most symptoms of Parkinson's disease. However, stimulation of the subthalamic nucleus may lead to a reduction in the dose of L-dopa after surgery, whereas stimulation of the medial globus pallidus may not lead to a reduction in the dose of L-dopa after surgery. The subthalamic nucleus has a good effect on Parkinson's disease tremor, and may be selected as the first location 111 and the second location 121 of the deep brain stimulation method for most patients with Parkinson's disease.

Here, the first position 111 and the second position 121 may be appropriate deep brain structures such as thalamus, basal ganglia, cerebral cortex, brainstem, and cerebellum that have effective therapeutic effects on brain and neural diseases such as tremor, myodystonia, Alzheimer's dementia, epilepsy, obsessive compulsive disorder, depression, pain, drug addiction, and post-traumatic syndrome.

The stimulator 130 may be connected to the first stimulation electrode 110 and the second stimulation electrode 120 to supply the current to the first stimulation electrode 110 and the second stimulation electrode 120.

The stimulator 130 is a nerve stimulator powered by an internally contained battery, and may be wired or wirelessly connected to and controlled by an external controller, and may supply a current from the battery to the first stimulation electrode 110 and the second stimulation electrode 120 at a desired frequency and at a desired magnitude. Both a rechargeable battery and a non-rechargeable battery may be used.

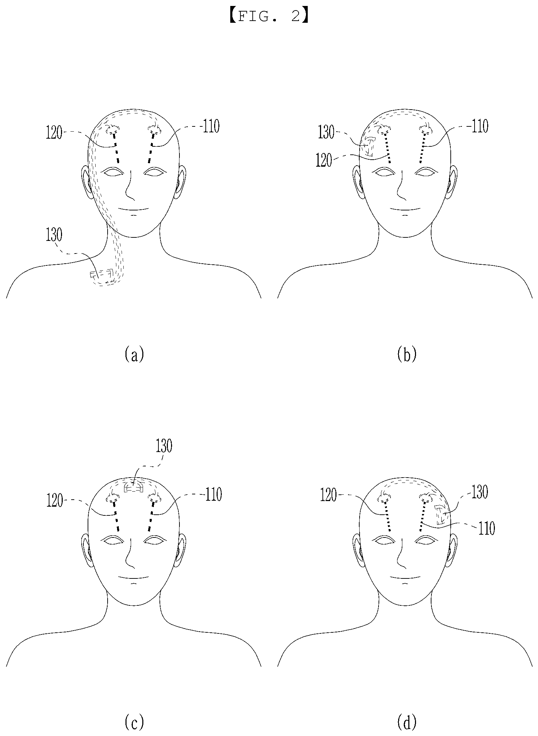

As shown in FIG. 2, the stimulator 130 may be located under the patient's collarbone, and may also be installed on the patient's head.

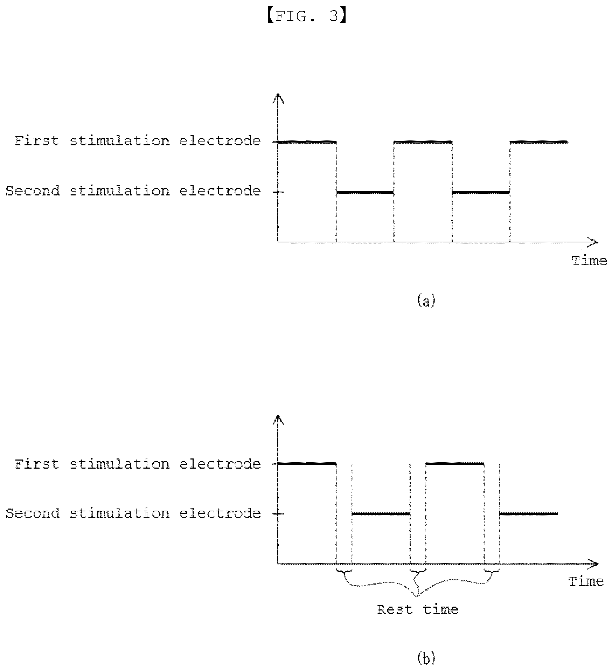

FIG. 3 is a schematic diagram illustrating a control method of a deep brain stimulation device according to an embodiment of the technical idea of the present disclosure.

The stimulator 130 is controlled so as not to simultaneously supply the currents to the first stimulation electrode 110 and the second stimulation electrode 120 constituting a pair of electrodes. Here, by controlled so as not to simultaneously supply the currents to the first and second stimulation electrodes constituting the pair of electrodes, it is meant that the current may be continuously supplied to only any one of these stimulation electrodes, or the currents may be alternately suppled to each of these stimulation electrodes. For example, the stimulator 130 may supply the currents to the first stimulation electrode 110 and the second stimulation electrode 120 by performing a current supply method comprising the following steps.

First, a step of not supplying the current to the second stimulation electrode 120 when supplying the current to the first stimulation electrode 110 during a first current supply time, may be performed.

Optionally, a step of not supplying the current to the first stimulation electrode 110 and supplying the current to the second stimulation electrode 120 during the second current supply time, when the first current supply time has elapsed, may be performed. Here, the "optionally" means that, in an extreme case, the current may be supplied to only any one of the first and second stimulation electrodes, and should be interpreted in the same manner hereinafter.

The above steps may be repeated, and the current supplied to the first stimulation electrode 110 and the current supplied to the second stimulation electrode 120 may have the same magnitude.

In addition, the current supply method of the stimulator 130 may further comprise: allowing the stimulator 130 to have a predetermined rest time before supplying the current to the second stimulation electrode 120, after supplying the current to the first stimulation electrode 110. During the predetermined rest time, no current is supplied to both the first stimulation electrode 110 and the second stimulation electrode 120 that constitute a pair.

With this configuration, it is possible to increase the life of a battery of the stimulator 130 by two or more times, while having treatment effects that are equivalent to or superior to those of a conventional method of simultaneously and continuously supplying the current to both stimulation electrodes that constitute a pair.

It is also possible to configure only first stimulation electrode 110 to be located at the first location 111 of the deep brain nucleus, or only the second stimulation electrode 120 to be located at the second location 121, and to configure the stimulator 130 to supply the current continuously or periodically with a predetermined rest time.

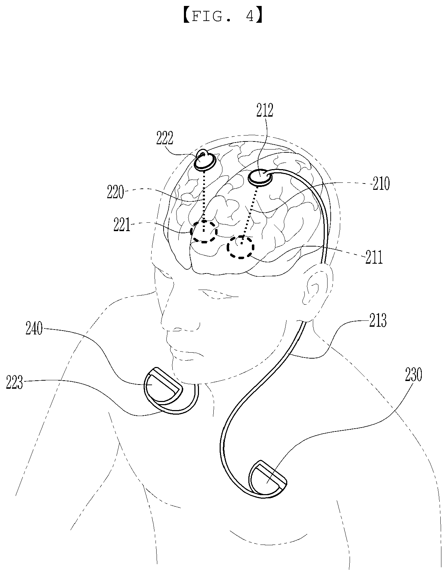

FIG. 4 is a drawing schematically illustrating a deep brain stimulation device according to another embodiment of the technical idea of the present disclosure.

A deep brain stimulation device according to another embodiment of the technical idea of the present disclosure may comprise a first stimulation electrode 210, a second stimulation electrode 220, a first stimulator 230, and a second stimulator 240.

The first stimulation electrode 210 may be located at a first location 211 of a deep brain nucleus, and the second stimulation electrode 220 may be located at a second location 221 of the deep brain nucleus. The first stimulation electrode 210 and the second stimulation electrode 220 may be long rod-shaped electrodes made of platinum or iridium, and may perform the function of applying electrical stimulation to the first location 211 and the second location 221 of the deep brain nucleus, respectively.

The first stimulation electrode 210 and the second stimulation electrode 220, which functionally constitute a pair of electrodes, may be fixed to a perforated skull through a first fixing part 212 and a second fixing part 222, respectively. The first stimulation electrode 210 may be connected to the first stimulator 230 through a first extension line 213 and the second stimulation electrode 220 may be connected to the first stimulator 230 through a second extension line 223, respectively, and the second stimulation electrode 220 may be connected to the second stimulator 240 via a subcutaneous passage through the second extension 223.

The first location 111 and the second location 121 may be the ventral intermediate nucleus of the thalamus, the medial globus pallidus, and the subthalamic nucleus, etc. The first location 111 and the second location 121 may be located so as to be symmetrical on both sides with respect to the center of the brain.

The first stimulator 230 may be connected to the first stimulation electrode 210 to supply a current to the first stimulation electrode 210, and the second stimulator 240 may be connected to the second stimulation electrode 220 to supply a current to the second stimulation electrode 220.

The first stimulator 230 and the second stimulator 240 are neuro stimulators powered by an internally contained battery, and may be wired or wirelessly connected to and controlled by an external controller, and may supply current from the battery to the first stimulation electrode 210 and the second stimulation electrode 220 at a desired frequency and at a desired magnitude. Both a rechargeable battery and a non-rechargeable battery may be used.

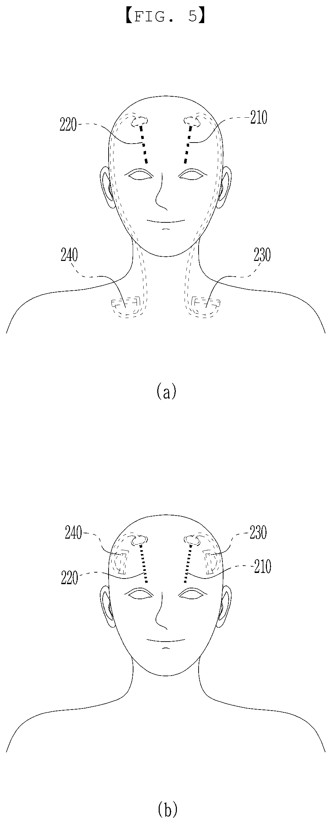

As shown in FIG. 5, the first stimulator 230 and the second stimulator 240 may be located under the patient's collarbone, and may also be installed on the patient's head.

The first stimulator 230 and the second stimulator 240 do not simultaneously supply the current to the first stimulation electrode 210 and the second stimulation electrode 220, which functionally constitute a pair of electrodes. For example, the first stimulator 230 and the second stimulator 240 may perform a current supply method including the following steps to supply the current to the first stimulator electrode 210 and the second stimulator electrode 220.

First, a step of the second stimulator 240 not supplying the current to the second stimulation electrode 220 when the first stimulator 230 supplies the current to the first stimulation electrode 210 during a first current supply time, may be performed.

Optionally, a step of the first stimulator 230 not supplying the current to the first stimulation electrode 210 and the second stimulator 240 supplying the current to the second stimulation electrode 220 during the second current supply time, when the first current supply time has elapsed, may be performed.

The above steps may be repeated, and the current supplied to the first stimulator electrode 210 and the current supplied to the second stimulator electrode 220 may have the same magnitude. Further, the first current supply time and the second current supply time may be the same as each other.

Here, after the first stimulator 230 supplies the current to the first stimulation electrode 210 during the first current supply time, the second stimulator 240 may supply the current to the second stimulation electrode 220 during the second current supply time after a predetermined rest time.

With this configuration, it is possible to increase the life of batteries of the first stimulator 230 and the second stimulator 240 by two or more times, while having treatment effects that are equivalent to or superior to those of a conventional method of simultaneously and continuously supplying the current to both stimulation electrodes.

The deep brain stimulation method according to an embodiment of the technical idea of the present disclosure may be may be applied to patients diagnosed with Parkinson's disease who exhibit dyskinesia or motor fluctuations due to long-term administration of L-dopa, or who experience side effects (gastrointestinal disorders, mental disorders) caused by overdose of L-dopa drugs, or patients with brain and neural diseases such as Parkinson's disease, tremor, myodystonia, Alzheimer's dementia, epilepsy, obsessive compulsive disorder, depression, pain, drug addiction, and post-traumatic syndrome, including patients with Parkinson's disease tremor that does not respond to L-dopa drugs.

The deep brain stimulation method according to an embodiment of the technical idea of the present disclosure may comprise: supplying the current to a first stimulation electrode located at a first location of the deep brain nucleus during a first current supply time, and supplying the current to a second stimulation electrode located at a second location of the deep brain nucleus during a second current supply time after a first current supply time. The above steps may be repeated.

Here, no current is supplied to the second stimulation electrode while the current is supplied to the first stimulation electrode, and no current is supplied to the first stimulation electrode while the current is supplied to the second stimulation electrode.

The current supplied to the first stimulation electrode and the current supplied to the second stimulation electrode may have the same magnitude, and after the current is supplied to the first stimulation electrode, the current may be supplied to the second stimulation electrode, when a predetermined rest time has elapsed.

Although the present disclosure has been described in detail above, the scope of the present disclosure is not limited thereto, and it will be apparent to those skilled in the art that various modifications and variations can be made without departing from the technical idea of the present disclosure as described in the claims.

## Claims

1. A deep brain stimulation device, comprising:
a first stimulation electrode configured to be located at a first location of a deep brain nucleus,
a second stimulation electrode configured to be located at a second location of the deep brain nucleus, and
a stimulator supplying currents to the first stimulation electrode and the second stimulation electrode,
wherein the stimulator is controlled so as not to supply the currents to the first stimulation electrode and the second stimulation electrode simultaneously.

2. The deep brain stimulation device of claim 1, wherein the stimulator is controlled to supply the current to only any one of the first and second stimulation electrodes.

3. The deep brain stimulation device of claim 1, wherein the stimulator is controlled to alternately supply the currents to each of the first and second stimulation electrodes.

4. The deep brain stimulation device of claim 1, wherein the stimulator performs a current supply method comprising:
not supplying the current to the second stimulation electrode when supplying the current to the first stimulation electrode during a first current supply time; and
not supplying the current to the first stimulation electrode and supplying the current to the second stimulation electrode during a second current supply time, when the first current supply time has elapsed.

5. The deep brain stimulation device of claim 4, wherein the current supply method further comprises:
allowing the stimulator to have a predetermined rest time before supplying the current to the second stimulation electrode, after supplying the current to the first stimulation electrode,
wherein during the predetermined rest time, no current is supplied to both the first stimulation electrode and the second stimulation electrode.

6. The deep brain stimulation device of claim 1, wherein the stimulator comprises:
a first stimulator connected to the first stimulation electrode and supplying the current to the first stimulation electrode; and
a second stimulator connected to the second stimulation electrode and supplying the current to the second stimulation electrode,
wherein the first stimulator and the second stimulator are controlled so as not to supply the currents to the first stimulation electrode and the second stimulation electrode simultaneously.

7. The deep brain stimulation device of claim 6, wherein a current supply method is performed, the method comprising:
the second stimulator not supplying the current to the second stimulation electrode when the first stimulator supplies the current to the first stimulation electrode during a first current supply time; and
the first stimulator not supplying the current to the first stimulation electrode and the second stimulator supplying the current to the second stimulation electrode during a second current supply time, when the first current supply time has elapsed.

8. The deep brain stimulation device of claim 1, wherein the current supplied to the first stimulation electrode and the current supplied to the second stimulation electrode have the same magnitude.

9. The deep brain stimulation device of claim 4 or 7, wherein the first current supply time and the second current supply time are the same as each other.

10. The deep brain stimulation device of claim 4 or 7, wherein the deep brain stimulation device allows the second stimulator to supply the current to the second stimulation electrode during the second current supply time after a predetermined rest time, after the first stimulator supplies the current to the first stimulation electrode during the first current supply time.

11. A control method of a deep brain stimulation device, comprising:
controlling currents to be supplied to each of a pair of stimulation electrodes configured to be located at predetermined locations of a deep brain nucleus,
wherein the currents are controlled not to be supplied to each of stimulation electrodes constituting the pair of stimulation electrodes simultaneously.

12. The control method of claim 11, wherein the current is controlled to be supplied to only any one of the stimulation electrodes constituting the pair of stimulation electrodes.

13. The control method of claim 11, wherein the currents are controlled to be alternately supplied to each of the pair of stimulation electrodes.

14. The control method of claim 11, further comprising:
supplying the current to a first stimulation electrode located at a first location of the deep brain nucleus during a first current supply time; and
supplying the current to a second stimulation electrode located at a second location of the deep brain nucleus during a second current supply time, after the first current supply time,
wherein the current is controlled so as not to be supplied to the second stimulation electrode while the current is supplied to the first stimulation electrode, and
the current is controlled so as not to be supplied to the first stimulation electrode while the current is supplied to the second stimulation electrode.

15. The control method of claim 14, wherein the current supplied to the first stimulation electrode and the current supplied to the second stimulation electrode have the same magnitude.

16. The control method of claim 14, wherein the first current supply time and the second current supply time are the same as each other.

17. The control method of claim 14, wherein after the current is supplied to the first stimulation electrode, the current is supplied to the second stimulation electrode, when a predetermined rest time has elapsed.
